# EUROPEAN PATENT SPECIFICATION

(11) **EP 0 419 164 B1**
(45) Date of publication and mention of the grant of the patent: **30.11.1994**
(21) Application number: 90310119.4
(22) Date of filing: 17.09.1990
(51) Int. Cl.: A61K 7/00, A61K 7/42

(54) **Hair conditioning composition**
Haarkonditionierungszusammensetzung
Composition pour le conditionnement des cheveux

(30) Priority: 18.09.1989 GB 8921074
(43) Date of publication of application: 27.03.1991
(73) Proprietor: UNILEVER PLC, London EC4P 4BQ (GB); UNILEVER N.V., 3013 AL Rotterdam (NL)
(72) Inventor: Birtwistle, David Howard, Wirral, Merseyside L61 9QN (GB); Marti, Vernon Peter John, Wirral, Merseyside L63 9YH (GB)
(74) Representative: Mulder, Cornelis Willem Reinier, Dr.

(56) References cited:
- EP-A- 0 012 178
- US-A- 4 567 038
- US-A- 4 810 489
- MANUFACTURING CHEMIST, vol. 58, no. 11, November 1987, pages 62-63, London, GB;"Out in the sun"

## Description

### BACKGROUND OF THE INVENTION

The present invention relates to hair conditioning compositions intended to be applied to hair and then rinsed off. More particularly, the invention relates to hair conditioning compositions containing sunscreen materials to protect the hair from the harmful effects of sunlight.

Dark hair exposed to sunlight fades, oriental black hair takes on a reddish tinge, and blond hair tends to turn yellow. The hair becomes rougher and drier to the touch after prolonged exposure to sunlight and also tends to lose its glossy appearance.

The hair can be protected from damage by exposure to sunlight by coating the hair fibers with an effective sunscreen.

Conventional hair conditioning compositions intended to be applied to hair and then rinsed off are generally made up of three phases, a first lamellar phase made up of cationic surfactant and long chain fatty alcohol, a second aqueous phase which is mainly water with some micellar cationic surfactant and a third fatty (oily) phase consisting of fatty alcohol and fatty thickeners, for example stearyl stearate, glyceryl monostearate or paraffin wax.

When oil-soluble sunscreen material is introduced into the above conventional hair conditioning composition it partitions preferentially into the fatty phase. When the composition is rinsed from the hair, the fatty phase which contains most of the sunscreen is washed away, and little sunscreen benefit is retained by the hair. Any sunscreen which is deposited is contained in fatty lumps and does not spread to give even protection over the whole of the hair shaft.

Water-soluble sunscreen would be washed away by the rinsing water in the final rinsing step of the conditioning treatment, and is therefore unsuitable for use in conditioning compositions.

Attempts have been made to overcome the problems of rinsing away sunscreen benefits from the hair. For example, US 4 104 368 (Millmaster) discloses hair and skin compositions for conditioning and protecting against UV damage, which comprise water-soluble compounds made up of long chain quaternary ammonium cations and acidic water-soluble sunscreen anions. The cationic moiety is thought to give the compounds some substantivity to skin and hair.

EP-A-193392 (Revlon) discloses a mousse product intended to be applied to hair and left on the hair (not rinsed off). It contains water-soluble sunscreen, or sunscreen dissolved in an aqueous alcohol phase.

Manufacturing Chemist, vol. 58, no 11, Nov. 1987, 62, 63, describes a sunscreen formulation (Chemviron) comprising an oil sunscreen material (Escalol 507), cetyl alcohol and Merquat S (Polyquaternium 7) which latter is not a surfactant and does not form a lamellar phase.

### BRIEF SUMMARY OF THE INVENTION

We have found that by effectively excluding the fatty phase from a hair conditioner composition and including a sunscreen which is an oil, the amount of sunscreen which is rinsed away can be substantially reduced and correspondingly the amount which remains available for retention on the hair is increased.

Broadly, the invention provides a hair conditioning composition comprising a lamellar phase, which lamellar phase comprises an oil sunscreen material.

More specifically, the invention provides a hair conditioning composition comprising a first, aqueous phase and a second, lamellar-phase including cationic surfactant, characterized in that the composition comprises an oil sunscreen material and the composition is in two phases with the oil sunscreen material present in the lamellar phase and that the level of free fatty material present in the composition is less than 0.4% by weight of the composition.

The composition may optionally further comprise an oil-soluble sunscreen material.

### DETAILED DESCRIPTION OF THE INVENTION

In the hair conditioning composition according to the invention, the level of fatty material able to form a third, fatty/oily phase is low, and the oil sunscreen material and the optional oil-soluble sunscreen material are found in the lamellar phase. It is thought that when the hair conditioning composition is applied to the hair, it is the lamellar phase which deposits onto and lubricates the hair. The sunscreen material is carried onto the hair by the lamellar phase and deposits in an even coating, giving uniform protection.

### Lamellar phase

The lamellar phase contains cationic surfactant. Generally it also contains long chain fatty alcohol.

The cationic surfactant will generally function as a hair conditioning agent (as will be mentioned again below) but other hair conditioning agents may be present in addition.

The amount of surfactant present in the composition will generally be from 0.01 to 5% by weight, preferably 0.1 to 5%, more preferably 0.1 to 1% by weight.

Examples of cationic surfactant include
Cetyl trimethylammonium chloride
Stearyl dimethylbenzyl ammonium chloride
Cetylpyridinium chloride
Quaternium-5
Quaternium-31
Quaternium-18
and mixtures thereof
Long chain fatty alcohol which is present may have fatty alkyl or alkenyl chains with 14 to 22 carbon atoms, preferably 16 to 20. Fatty alcohol which is particularly suitable for use in conjunction with cationic surfactant is a mixture of cetyl and stearyl alcohols, customarily referred to as "cetostearyl alcohol".

The use of fatty alcohol with cationic surfactant is a known technique for retaining cationic surfactant in lamellar phase. The amount of fatty alcohol is desirably the minimum required for this purpose plus a slight excess. Any more fatty alcohol is undesirable for this invention as it will contribute to a separate third phase. The appropriate weight ratio can be determined by making mixtures in various proportions and examining them for free fatty material as described below. For cetyl trimethyl ammonium chloride used in conjunction with a mixture of cetyl and stearyl alcohols, a suitable weight ratio of cationic surfactant to fatty alcohol is in the range 1:1.3 to 1:2 preferably 1:1.3 to 1:1.6.

### Sunscreen materials

The hair conditioning composition of the invention comprises an oil sunscreen material, preferably in an amount of from 0.1 to 10% by weight, most preferably from 0.2 to 2% by weight.

Examples of suitable oil sunscreen materials are 2-ethylhexyl-p-methoxy Cinnamate, sold commercially as Parsol MCX, 2-ethoxyethyl-p-methoxy cinnamate, sold commercially as Giv-Tan F, isoamyl-p-methoxycinnamate, sold commercially as Neo-Heliopan E1000, 2-ethylhexyl-p-dimethylaminobenzoate sold commercially as Escalol 507 and ethyl dihydroxypropyl-p-aminobenzoate sold commercially as Amerscreen P.

The lamellar phase of the hair conditioning compositions of the invention preferably further comprises a sunscreen material which is not itself an oil but is oil-soluble. Such materials will generally be oil-soluble solids and suitably are used in an amount of from 0.1 to 8% by weight, preferably from 0.2 to 2% by weight.

Examples of preferred oil-soluble sunscreens are benzophenone derivatives such as 4,4′-tetrahydroxy-benzophenone, sold commercially as Uvinul D50, and 2-hydroxy-4-methoxy-benzophenone, sold commercially as Eusolex 4360, dibenzoyl methane derivatives such as t-butyl-4-methoxydibenzoyl methane, sold commercially as Parsol 1789, and isopropyldibenzoyl methane, sold commercially as Eusolex 8020.

When the composition comprises oil-soluble sunscreen, the oil-soluble sunscreen dissolves in the oil sunscreen and is contained in the lamellar phase. It is then retained on the hair together with the oil sunscreen.

It is preferred that the total amount of sunscreen material, i.e. oil sunscreen material and oil-soluble sunscreen material, in the hair conditioning composition does not exceed 10% by weight. Little increase in protection is seen when the composition contains over 10% by weight of sunscreen material, and the hair tends to take on a greasy appearance.

### Free fatty material

The hair conditioning composition of the invention is essentially a two-phase composition which contains very low levels of free fatty material. In this context, "free fatty material" means fatty material which is not bound up in the lamellar phase and can form a third, fatty/oily phase. The term excludes the sunscreen material. The amount of free fatty material is sufficiently low, less than 0.4% by weight of the composition, that the oil sunscreen material is present in the lamellar phase.

The amount of free fatty material in a hair conditioner composition can be detected upon ultracentrifugation at approximately 36000 rpm for 2 hours. The composition separates into layers and any free fatty material present will form the least dense layer which rises to the top. The next layer is the lamellar phase, and in the composition of the invention which contains essentially no fatty phase, it is this lamellar phase which contains most of the sunscreen material. The remaining liquid layer consists essentially of water.

On centrifugation of a conventional hair conditioning composition comprising cationic surfactant, long chain fatty alcohol and fatty thickeners the sunscreen material partitions such that the fatty layer contains about 39% by weight of the total amount of sunscreen present in the composition, the lamellar phase contains about 49.7% by weight and the aqueous liquid phase contains about 11.3% by weight.

The hair conditioning composition of the invention which contains substantially no fatty phase partitions on centrifugation such that the lamellar phase contains about 81.5% by weight of the total amount of sunscreen and the aqueous liquid phase contains about 18.5% by weight.

It can be seen that the amount of unbound sunscreen available for coating the hair fibers is greatly increased in the composition of the invention.

Generally the total amount of fatty material present in the composition and which is capable of contributing to formation of a separate fatty/oily third phase containing at least part of that fatty material is less than 5% by weight of the composition, generally well below 5%.

If the hair conditioning composition of the invention does contain low levels of free fatty material, this will be due mainly to perfumes and other minor ingredients which are fatty in nature.

### Conditioning Agents

The cationic surfactant included in compositions of this invention serves as a hair conditioning agent. The compositions may also comprise other conditioning agents which may be chosen from cationic polymers, involatile silicones, volatile silicones, protein hydrolysates or quaternised protein hydrolysates.

Suitable cationic polymers include
Guar Hydroxypropyltrimonium chloride
Quaternium-19
Quaternium-23
Quaternium-40
Quaternium-57
Poly (dimethyldiallylammonium chloride)
Poly (dimethylbutenyl ammonium chloride)-α,ω-bis (triethanolammonium chloride)
Poly (dipropyldiallylammonium chloride)
Poly (methyl-β-propaniodiallylammonium chloride)
Poly (diallylpiperidinium chloride)
Poly (vinyl pyridinium chloride)
Quaternised poly (vinyl alcohol)
Quaternised poly (dimethylaminoethylmethacrylate) and mixtures thereof.

Examples of suitable volatile silicone materials include cyclomethicone, available commercially as Dow Corning DC 345, and Volatile Silicone 7158, available from Union Carbide.

The involatile silicones which may be used in the compositions of the invention are generally amino functional polydimethylsiloxanes. Examples include amodimethicone available commercially as Dow Corning DC 929 and trimethylsilylamodimethicone, available as Dow Corning Q 8220.

Suitable protein derivatives include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

The hair conditioning composition of the invention preferably comprises from 0.01 to 5% by weight, most preferably from 0.1 to 1% by weight of conditioning agent(s) including the cationic surfactant.

### Other ingredients

The hair conditioning composition of the invention may be thickened by means of a neutral polymer. Suitable thickening agents include guar gum, available as Jaguar Gum or Jaguar Plus, hydroxypropyl guar, available as Jaguar HP 60, xanthan gum, available as Kelzan, and hydroxyethylcellulose, available as Natrosol 150 or Natrosol 250HR.

The composition may also comprise minor amounts of other ingredients which are commonly employed in hair conditioners. Examples of such ingredients include viscosity-adjusting agents, opacifiers, pearlescers, perfumes, colouring agents, preservatives and buffering agents.

### Examples

The invention is further illustrated by the following Examples.

### Example 1

| | % wt |
|---|---|
| Cetostearyl alcohol | 0.94 |
| Cetyl trimethylammonium chloride (50% aqueous solution) | 1.4 |
| Natrosol 250 HR | 1.25 |
| Polystyrene latex | 1.5 |
| Parsol MCX | 1.0 |
| Perfume, preservative | qs |
| Water | to 100 |

The Natrosol 250 HR is dissolved in water and heated to 80°C. Cetostearyl alcohol and cetyl trimethylammonium chloride are mixed together and added to the Natrosol 250 HR solution with mixing. The mixture is cooled with stirring, and the remaining ingredients, except Parsol MCX are added. Finally Parsol MCX is added with stirring.

The following hair conditioning compositions may be made by following the method outlined in Example 1 above.

### Example 2

| | % wt |
|---|---|
| Cetostearyl alcohol | 1.01 |
| Cetyl trimethylammonium chloride (50% aqueous solution) | 1.50 |
| Jaguar HP60 | 1.0 |
| Escalol 507 | 2.0 |
| Eusolex 4360 | 0.5 |
| Polystyrene latex | 1.4 |
| Perfume, preservative | qs |
| Water | to 100 |

### Example 3

| | % wt |
|---|---|
| Cetostearyl alcohol | 0.81 |
| Cetyl trimethylammonium chloride (50% aqueous solution) | 1.20 |
| Natrosol HHXR | 1.06 |
| Neo Heliopan E1000 | 2.5 |
| Parsol 1789 | 0.5 |
| Perfume, preservative | qs |
| Water | to 100 |

### Example 4

| | % wt |
|---|---|
| Cetostearyl alcohol | 1.21 |
| Cetyl trimethylammonium chloride (50% aqueous solution) | 1.80 |
| Natrosol 250HR | 1.2 |
| Amerscreen P | 1.5 |
| Eusolex 4360 | 0.3 |
| Polystyrene latex | 1.0 |
| Perfume, preservative | qs |
| Water | to 100 |

### Example 5

| | % wt |
|---|---|
| Cetostearyl alcohol | 0.94 |
| Cetyl trimethylammonium chloride (50% aqueous solution) | 1.40 |
| Jaguar C-13-5 | 1.0 |
| Parsol MCX | 3.0 |
| Eusolex 8020 | 0.75 |
| Perfume, preservative | qs |
| Water | to 100 |

## Claims

1. A hair conditioning composition comprising a first, aqueous phase and a second, lamellar phase including cationic surfactant, characterised in that the composition comprises an oil sunscreen material which material is present in the lamellar phase and that the level of free fatty material present in the composition is less than 0.4% by weight of the composition.

2. A hair conditioning composition as claimed in claim 1 wherein the oil sunscreen material is present in an amount of from 0.1 to 10% by weight.

3. A hair conditioning composition as claimed in claims 1 or 2 wherein the lamellar phase further contains an oil-soluble sunscreen material.

4. A hair conditioning composition as claimed in claim 3 wherein the oil-soluble sunscreen material is present in an amount of from 0.1 to 8% by weight.

5. A hair conditioning composition as claimed in any preceding claim wherein the total amount of sunscreen material in the composition does not exceed 10% by weight.

6. A hair conditioning composition as claimed in any preceding claim wherein the oil sunscreen material is chosen from 2-ethylhexyl-p-methoxycinnamate,
2-ethoxyethyl-p-methoxycinnamate,
isoamyl-p-methoxycinnamate,
2-ethylhexyl-p-dimethylaminobenzoate and
ethyl dihydroxypropyl-p-aminobenzoate.

7. A hair conditioning composition as claimed in claim 3 wherein the oil-soluble sunscreen material is chosen from 4,4'-tetrahydroxy-benzophenone,
2-hydroxy-4-methoxy-benzophenone,
t-butyl-4-methoxydibenzoyl methane and isopropyldibenzoyl methane.

8. A hair conditioning composition as claimed in any preceding claim wherein the composition comprises less than 5% by weight of fatty material able to form a separate fatty/oily third phase comprising at least part of that fatty material.

9. A hair conditioning composition as claimed in any preceding claim, comprising a hair conditioning agent other than the cationic surfactant.

## Patentansprüche

1. Haarkonditionierzusammensetzung mit einer ersten wäßrigen Phase und einer zweiten lamellaren Phase, einschließlich eines kationischen Netzmittels, dadurch gekennzeichnet, daß die Zusammensetzung ein in der lamellaren Phase vorliegendes Ölsonnenfiltermaterial umfaßt, und dadurch, daß der Gehalt an dem in der Zusammensetzung vorhandenen freien Fettmaterial weniger als 0,4 Gew.-% der Zusammensetzung beträgt.

2. Haarkonditionierzusammensetzung nach Anspruch 1, wobei das Ölsonnenfiltermaterial in einer Menge von 0,1 bis 10 Gew.-% vorhanden ist.

3. Haarkonditionierzusammensetzung nach Ansprüchen 1 oder 2, wobei die lamellare Phase des weiteren ein öllösliches Sonnenfiltermaterial enthält.

4. Haarkonditionierzusammensetzung nach Anspruch 3, wobei das öllösliche Sonnenfiltermaterial in einer Menge von 0,1 bis 8 Gew.-% vorhanden ist.

5. Haarkonditionierzusammensetzung nach einem der vorhergehenden Ansprüche, wobei die Gesamtmenge des Sonnenfiltermaterials in der Zusammensetzung 10 Gew.-% nicht übersteigt.

6. Haarkonditionierzusammensetzung nach einem der vorhergehenden Ansprüche, wobei das Ölsonnenfiltermaterial aus 2-Ethylhexyl-p-methoxycinnamat, 2-Ethoxyethyl-p-methoxycinnamat, Isoamyl-p-methoxycinnamat, 2-Ethylhexyl-p-dimethylaminobenzoat und Ethyldihydroxypropyl-p-aminobenzoat ausgewählt ist.

7. Haarkonditionierzusammensetsung nach Anspruch 3, wobei das öllösliche Sonnenfiltermaterial aus 4,4'-Tetrahydroxybenzophenon, 2-Hydroxy-4-methoxy-benzophenon, tert-Butyl-4-methoxydibenzoylmethan und Isopropyldibenzoylmethan ausgewählt ist.

8. Haarkonditionierzusammensetzung nach einem der vorhergehenden Ansprüche, wobei die Zusammensetzung weniger als 5 Gew.-% Fettmaterial mit der Fähigkeit zur Bildung einer getrennten dritten Fett/Öl-Phase mit mindestens einem Teil des Fettmaterials umfaßt.

9. Haarkonditionierzusammensetzung nach einem der vorhergehenden Ansprüche, umfassend ein sich von dem kationischen Netzmittel unterscheidendes Haarkonditioniermittel.

## Revendications

1. Une composition pour le conditionnement des cheveux comprenant une première phase aqueuse et une seconde phase lamellaire contenant un agent tensioactif cationique et caractérisée en ce que la composition contient une matière d'écran solaire huileuse, laquelle matière est présente dans la phase lamellaire, et en ce que la proportion de matière grasse libre présente dans la composition est inférieure à 0,4 % en masse de la composition.

2. Une composition pour le conditionnement des cheveux selon la Revendication 1, dans laquelle la matière d'écran solaire huileuse est présente dans une proportion allant de 0,1 à 10 % en masse.

3. Une composition pour le conditionnement des cheveux selon l'une quelconque des revendications 1 ou 2, dans laquelle la phase lamellaire comprend en outre une matière d'écran solaire soluble dans l'huile.

4. Une composition pour le conditionnement des cheveux selon la Revendication 3, dans laquelle la matière d'écran solaire soluble dans l'huile est présente dans une proportion allant de 0,1 à 8 % en masse.

5. Une composition pour le conditionnement des cheveux selon l'une quelconque des Revendications précédentes, dans laquelle la proportion totale de matière d'écran solaire dans la composition n'excède pas 10 % en masse.

6. Une composition pour le conditionnement des cheveux selon l'une quelconque des Revendications précédentes, dans laquelle la matière d'écran solaire huileuse est choisie parmi le 2-éthylhexyl-p-méthoxycinnamate, le 2-éthoxyéthyl-p-méthoxycinnamate, l'isoamyl-p-méthoxycinnamate, le 2-éthylhexyl-p-diméthylaminobenzoate et l'éthyldihydroxypropyl-p-aminobenzoate.

7. Une composition pour le conditionnement des cheveux selon la Revendication 3, dans laquelle la matière d'écran solaire soluble dans l'huile est choisie parmi le 4,4'-tétrahydroxybenzophénone, le 2-hydroxy-4-méthoxybenzophénone, le t-butyl-4-méthoxydibenzoylméthane et l'isopropyldibenzoylméthane.

8. Une composition pour le conditionnement des cheveux selon l'une quelconque des Revendications précédentes, dans laquelle la composition contient moins de 5 % en masse de matière grasse pouvant former une troisième phase séparée grasse/huileuse comprenant au moins une partie de cette matière grasse.

9. Une composition pour le conditionnement des cheveux selon l'une quelconque des Revendications précédentes, comprenant un agent de conditionnement pour les cheveux autre que l'agent tensioactif cationique.
